# EUROPEAN PATENT SPECIFICATION

(11) **EP 4 444 202 B1**
(45) Date of publication and mention of the grant of the patent: **26.11.2025**
(21) Application number: 22830675.9
(22) Date of filing: 22.11.2022
(51) Int. Cl.: A61B 17/72, A61B 17/70, A61B 17/00, A61B 17/68

(54) **ADJUSTABLE IMPLANT WITH CYCLOID GEARS**
EINSTELLBARES IMPLANTAT MIT ZYKLOIDEN ZAHNRÄDERN
IMPLANT RÉGLABLE À ENGRENAGES CYCLOÏDES

(30) Priority: 07.12.2021 US 202163286699 P
(43) Date of publication of application: 16.10.2024
(73) Proprietor: NuVasive Specialized Orthopedics, Inc., San Diego, CA 92121 (US)
(72) Inventor: LOPEZ CAMACHO, Jorge, San Diego, CA 92121 (US)
(74) Representative: Morabito, Sara
(86) International application number: PCT/US2022/080321
(87) International publication number: WO 2023/107823

(56) References cited:
- EP-A1- 2 915 496
- WO-A2-2011/029021
- WO-A2-2016/134326
- US-A1- 2023 041 121

## Description

### CROSS REFERENCE TO RELATED APPLICATION

The present international patent application claims priority to US provisional patent application no. 63/286,699, filed 07 December 2021.

### BACKGROUND OF THE INVENTION

The present disclosure relates generally to distraction devices, and more particularly, to distraction devices that are scalable to small implant sizes.

Limb length discrepancy (LLD) is a condition in which a subject may have limbs of unequal length, caused by unequal lengths of one or more bones of the limb, such as the femur, tibia, or both. LLDs can be present at birth, or they can develop during an individual's lifetime, e.g., as a result of a fracture or a traumatic injury. LLDs can also be caused by infections, tumors, and may be the result of chronic fractures, congenital abnormalities, or non-unions. Depending on the severity of the LLD, non-surgical or surgical treatments may be appropriate. Non-surgical treatments may include, e.g., mere observation or the use of shoe lift. Surgical treatments suitable for more severe cases may include procedures including epiphysiodesis, limb shortening, external limb lengthening, or internal limb lengthening.

Intra- and extramedullary limb lengthening implants have been described in, e.g., International Patent Application Publication Nos. WO 2011/029021 A1, WO2016/134326 A2 and WO/2017/132646 A1. However, intramedullary and extramedullary implants are desirable that can be scaled down to accommodate the needs of smaller patients, and conditions involving the lengthening of smaller bones.

### BRIEF DESCRIPTION OF THE INVENTION

The invention is defined in claim 1. Further embodiments of the invention are recited in the dependent claims. A first aspect of the disclosure provides a device configured for placement between a first section of a bone and a second section of the bone. The device comprises a distraction shaft having an internal cavity disposed therein, the distraction shaft being configured for fixation to the first section of bone; and a housing configured for fixation to the second bone section. The distraction shaft is configured to be axially movable relative to, and disposed partially within the housing. The device further comprises a driving element disposed within the housing; a cycloid gear assembly configured to be rotatably driven by the driving element; and a lead screw assembly disposed at least partly within the internal cavity of the distraction shaft. The lead screw assembly is configured to rotatably advance and/or retract a lead screw within the internal cavity, and to be rotatably driven by the cycloid gear assembly.

A second aspect of the disclosure provides a device configured for placement between a first section of a bone and a second section of the bone. The device comprises a distraction shaft having an internal cavity disposed therein, and a first tab protrusion extending from a shoulder adjacent an open end of the internal cavity. The distraction shaft is configured for fixation to the first section of bone. The device further includes a housing configured for fixation to the second bone section, wherein the distraction shaft is configured to be axially movable relative to, and disposed partially within the housing. A driving element is disposed within the housing; and a gear assembly is configured to be rotatably driven by the driving element. A lead screw assembly is further configured to rotatably engage with the internal cavity, and to be rotatably driven by the gear assembly. In such embodiments, the lead screw assembly comprises: a lead screw disposed at least partly within the internal cavity of the distraction shaft; and an anti-jam feature disposed over the lead screw, between the open end of the distraction shaft and the gear assembly, wherein the anti-jam feature comprises a helical spring having second tab protrusion configured to matingly engage with the first tab protrusion on the distraction shaft.

A third aspect of the disclosure provides a device configured for placement between a first section of a bone and a second section of the bone, the device comprising: a distraction shaft having an internal cavity disposed therein, wherein the distraction shaft has an oblong cross sectional shape, and is configured for fixation to the first section of bone; and a housing configured for fixation to the second bone section. The distraction shaft is configured to be axially movable relative to, and disposed partially within the housing. A driving element is disposed within the housing; and a gear assembly is configured to be rotatably driven by the driving element. A lead screw assembly is disposed at least partly within the internal cavity of the distraction shaft, the lead screw assembly being configured to rotatably advance or retract a lead screw within the internal cavity, and to be rotatably driven by the gear assembly.

These and other aspects, advantages and salient features of the invention will become apparent from the following detailed description, which, when taken in conjunction with the annexed drawings, where like parts are designated by like reference characters throughout the drawings, disclose embodiments of the invention.

### BRIEF DESCRIPTION OF THE DRAWINGS

FIG. 1 illustrates a side view of an intramedullary lengthening device in place within a bone.
FIG. 2 shows a top view of a device including a distraction rod and a housing, in accordance with an embodiment of the invention.
FIG. 3 shows a side view of a device including a distraction rod and a housing, in accordance with an embodiment of the invention.
FIG. 4 shows a side view of a distraction rod as shown in FIG. 3 with the housing removed, in accordance with an embodiment of the invention.
FIG. 5 shows a cross sectional side view of a device including a distraction rod and a housing, in accordance with an embodiment of the invention.
FIG. 6 shows a cross sectional top view of a device including a distraction rod and a housing, in accordance with an embodiment of the invention.
FIG. 7 shows an exploded side view of aspects of the gear and magnet assembly of the device, in accordance with an embodiment of the invention.
FIG. 8 shows an exploded perspective view of aspects of the gear and magnet assembly of the device, in accordance with an embodiment of the invention.
FIG. 9 shows an exploded perspective view of aspects of the gear and magnet assembly of the device, in accordance with an embodiment of the invention.
FIG. 10 shows a side view of a lead screw as shown in FIGS. 5-6, in accordance with an embodiment of the invention.
FIG. 11 shows an exploded perspective view of aspects of the gear and magnet assembly of the device, in accordance with an embodiment of the invention.
FIG. 12 shows a perspective view of an eccentric magnet cup, in accordance with an embodiment of the invention.
FIGS. 13A-13C show perspective views of aspects of an anti-jam feature in accordance with an embodiment of the invention.
FIGS. 14A and 14B shows a perspective view and a cross sectional view, respectively, of an oblong nut in accordance with an embodiment of the invention.
FIG. 15 shows a perspective view of aspects of a distraction rod in accordance with an embodiment of the invention.
FIG. 16 shows a cross sectional view of the distraction rod of FIG. 15, in accordance with an embodiment of the invention.
FIG. 17 illustrates a side view of a device affixed to vertebrae of a spine of an individual suffering from scoliosis, in accordance with an embodiment of the invention.

It is noted that the drawings of the disclosure are not necessarily to scale. The drawings are intended to depict only typical aspects of the disclosure, and therefore should not be considered as limiting the scope of the disclosure. In the drawings, like numbering represents like elements between the drawings.

### DETAILED DESCRIPTION OF THE INVENTION

At least one embodiment of the present invention is described below in reference to its application in connection with an extramedullary lengthening device. Although embodiments of the invention are illustrated relative to an extramedullary lengthening device, it is understood that the teachings are equally applicable to other implants including, but not limited to, intramedullary lengthening devices, intramedullary and extramedullary devices for performing compression of bones, and adjustable spinal rods configured to be coupled to vertebrae of a spine, e.g., via pedicle screws.

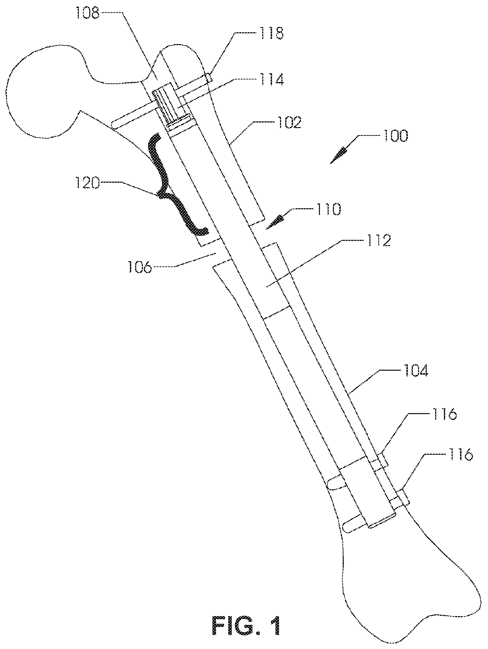

FIG. 1 illustrates a side view of a device 110, which is an intramedullary lengthening device which has been placed through a hole or bore 108 contained within a bone 100. The hole or bore 108 may be made by drilling, reaming and the like and may extend through both cortical bone (at the end) and through cancellous (spongy) bone. As illustrated in FIG. 1, device 110 includes a housing 112 and a distraction shaft 114. Bone 100 may either have a pre-existing separation 106 or may be purposely cut or broken to create this separation 106, dividing the bone into a first section 102 and a second section 104. The cut may be done prior to inserting and securing the device 110, or may be done after the device 110 is inserted, for example by use of a flexible Gigli saw. The distraction shaft 114 of the device 110 is attached to the first section 102 using one or more fasteners 118, which may include, e.g., screws as known to those skilled in the art. The housing 112 of the device 110 is secured to the second section 104 of bone 100 using one or more fasteners 116. Again, fasteners 116 may include screws or other types of fasteners as known in the art.

In order to grow or lengthen bone 100, over the treatment period the bone 100 is continually distracted. This enlarges or widens separation 106, within which osteogenesis can occur. "Continually distracted" is meant to indicate that distraction occurs on a regular basis which may be on the order of semi-daily, daily, or every few days. An exemplary distraction rate may be, e.g., one millimeter per day although other distraction rates may be employed. That is to say, a typical distraction regimen may include a daily increase in the length of the device 110 by about one millimeter. This may be done, for example, by four lengthening periods per day, each having 0.25 mm of lengthening. Device 110 may include a drive system configured to telescopically extend the distraction shaft 114 from the housing 112, thus forcing the first section 102 and the second section 104 of the bone 100 apart from one another. As the distraction is performed, a portion of the housing 112 may be configured to slide within the hole or bore 108 of the first section 102 of bone 100 within a displacement section 120.

In various embodiments, the orientation of the device 110 within the bone 100 may be opposite of that shown in FIG. 1. For example, the distraction shaft 114 may be coupled to the second section 104 of the bone 100 and the housing 112 may be coupled to the first section 102 of the bone 100. The device 110 may thus be placed in a retrograde orientation, from a hole or bore starting at the distal end of the bone 100.

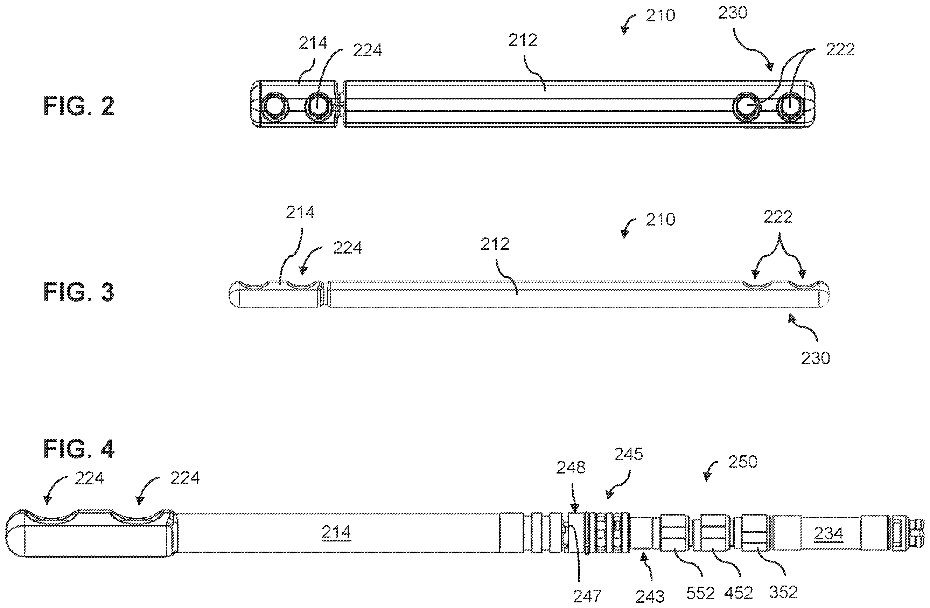

In other embodiments, the device may take other forms. For example, in certain embodiments such as those depicted in FIGS. 2-6, the device 210 may be in the form of an extramedullary device 210, configured use for either distraction or compression of bone as described in International Patent Application No. PCT/US2021041701. Device 210 is configured for placement between a first section of a bone and a second section of the bone, analogously to first and second sections 102, 104 of bone 100 as shown in FIG. 1. Device 210 includes a housing 212 and a distraction shaft 214. Housing 212 may include a sleeve portion which may have a cross sectional shape that is substantially annular or oblong. The sleeve portion may be attached to an end portion 230 which includes one or more fixation apertures or holes 222 configured to receive fasteners such as fasteners 116 (FIG. 1). End portion 230 may include, e.g., a plate, which may be curved, angled, or contoured in accordance with the particular size and shape of the bone to which it will be affixed. End portion 230 may also include an end cap. Distraction shaft 214, illustrated apart from housing 212 in FIG. 4, may include holes 224 configured to receive fasteners such as fasteners 118 (FIG. 1) as known in the art for fixation of the distraction shaft 214 to a section of bone. In various embodiments, holes 222 and 224 may be internally threaded, and may be configured to receive fasteners with complementary thread patterns to the thread patterns of holes 222, 224.

As described above with respect to device 110, device 210 is configured to telescopically extend and retract the distraction shaft 214 from the housing 212, thus forcing the first section and second sections of bone apart from one another upon extension, or drawing the first section and second sections together upon retraction. Distraction shaft 214 is configured to be at least partially inserted into housing 212 as shown in FIGS. 2-3, with the end of distraction shaft 214 opposite screw holes 222 being inserted into housing 212. In a shortest, most retracted configuration of device 210, distraction shaft 214 is disposed in significant part within the sleeve portion of housing 212. Distraction shaft 214 is telescopically movable relative to housing 212; in particular, distraction shaft 214 may translate axially in a sliding fashion into and out of housing 212.

Distraction shaft 214 may be configured for fixation to the first section 102 of bone 100 (FIG. 1) as previously described, while housing 212 may be configured for fixation to the second section 104 of bone 100 (FIG. 1). However, as described with respect to FIG. 1, a retrograde orientation of device 210 is also considered within the scope of the disclosure. As also described with respect to FIG. 1, in order to grow or lengthen the bone in which device 210 is implanted, over a treatment period the bone is continually distracted, thereby enlarging or widening the separation between the first and second sections of the bone within which osteogenesis occurs.

Device 210 includes a driving element disposed within the housing 212. In one embodiment, shown in FIGS. 4-6, the driving element may include a permanent magnet 234. Permanent magnet 234 may be cylindrical or substantially cylindrical in shape, and may be rotatable about a longitudinal axis of the cylinder within and relative to the housing 212. Such rotation may be magnetically driven by an external adjustment device as described in WO 2011/029021.

Although a number of embodiments are described herein in which the driving element is a permanent magnet 234, in other embodiments the driving element may take other forms such as, e.g., a motor or micromotor, and these embodiments are equally considered to be part of the present disclosure.

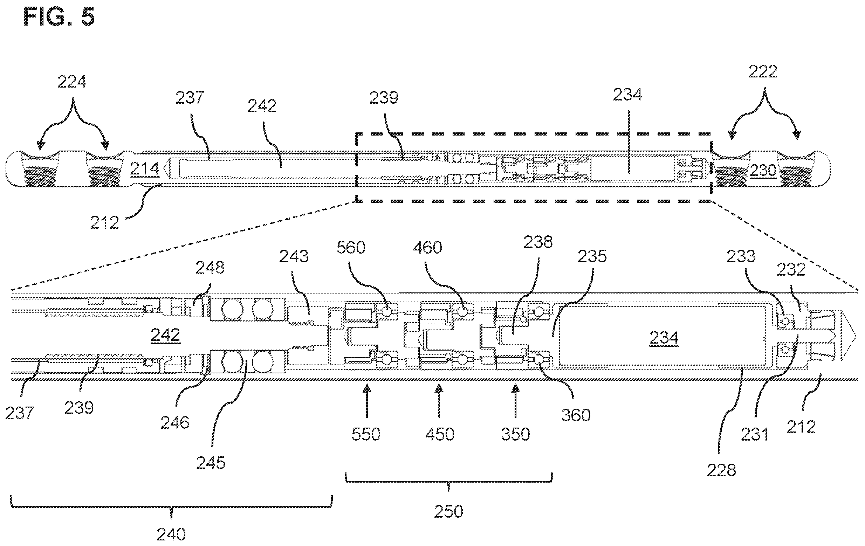

Permanent magnet 234 or another driving element as disclosed herein, may be rotationally coupled to a lead screw assembly 240 including lead screw 242. As shown in FIGS. 5-6, the lead screw assembly 240 is disposed at least partly within an internal cavity 237 within distraction shaft 214 (FIGS. 5, 6, 15, 16), where the lead screw assembly 240 is configured to rotatably advance and/or retract a lead screw 242 within the internal cavity 237. In use, rotation of the permanent magnet 234 causes rotation of lead screw 242, which rotates within a nut 239 that is secured to an inner surface of cavity 237 within distraction shaft 214. This rotation of lead screw 242 within nut 239 causes either advancement or retraction of lead screw 242 depending on the direction, i.e., clockwise or counterclockwise, of the rotation. Where the rotation of lead screw 242 causes its advancement in a direction further into cavity 237 of distraction shaft 214 and away from end portion 230, this causes distraction shaft 214 to be telescopically driven from housing 212. This in turn extends the length of device 210, and distracts the bone in which device 210 is implanted. Where rotation of lead screw 242 causes its retraction in a direction shallower in cavity 237 of distraction shaft 214 and toward end portion 230 of housing 212, this causes distraction shaft 214 to be drawn into housing 212, thereby compressing the bone in which device 210 is implanted.

As illustrated in FIGS. 5-6, permanent magnet 234 may be disposed within housing 212, and may be maintained in place on one end by a cap or cup 228 having a centric spindle 231 extending therefrom. Keeper 232 and bearing 233 cooperate with one another and with spindle 231 to support permanent magnet 234 within housing 212, and to achieve and maintain proper axial stack height during rotation of permanent magnet 234. Centric spindle 231 may extend through bearing 233 and keeper 232 near the end of the housing 212, around which permanent magnet 234 may rotate when actuated, e.g., by an external adjustment device.

On the opposite end of permanent magnet 234, a coupling member in the form of, e.g., a cup or cap 235 may be disposed over an end portion of permanent magnet 234. Cap 235 may include an eccentric shaft 238 extending therefrom in a direction opposite spindle 231, and toward lead screw 242 and distraction shaft 214. Bearing 360 may be provided to support eccentric shaft 238 in use. Cap 235 with eccentric shaft 238 may couple the driving element, e.g. permanent magnet 234 to the gear assembly. In certain embodiments disclosed herein, the gear assembly may particularly be a cycloid gear assembly 250. Cap 235 is further configured to be rotated along with permanent magnet 234, and, using the eccentric shaft 238, to rotatably drive the cycloid gear assembly 250. Further details of the engagement of permanent magnet 234, cap 235 and eccentric shaft 238, and cycloid gear assembly 250 are described further below.

With continued reference to FIGS. 5-6, in various embodiments described herein, permanent magnet 234 or other driving element may be coupled to lead screw assembly 240 by a cycloid gear assembly 250. Cycloid gear assembly 250 may be configured to be rotatably driven by permanent magnet 234, and to in turn rotate lead screw 242. In particular, magnet cup 235 with eccentric shaft 238 may engage cycloid gear assembly 250. Permanent magnet 234, cycloid gear assembly 250, and lead screw 242 are all configured to rotate relative to housing 212 and distraction shaft 214, thereby producing axial movement of lead screw 242 within internal cavity 237 as discussed above, thereby lengthening or shortening the device 210, and without causing rotation of, e.g., distraction shaft 214 within housing 212.

With reference to FIGS. 5-7, cycloid gear assembly 250 may include one or more gear stages, for example, one, two, three, four, or more than four gear stages. In the examples depicted, cycloid gear assembly 250 includes three stages, including first stage 350, second stage 450, and third stage 550. In certain embodiments, each stage 350, 450, 550, and so on, of the one or more stages within the cycloid gear assembly 250 may be configured to provide a particular gear reduction ratio. For example, each gear stage 350, 450, 550, and so on may provide a gear reduction ratio of 4:1. Thus, a cycloid gear assembly having two stages may provide a gear reduction ratio of 16:1, and a cycloid gear assembly having three stages may provide a gear reduction ratio of 64:1.

As described herein with respect to the accompanying figures, components of the first stage 350, second stage 450, and third stage 550 of cycloid gear assembly 250 will use 300 series, 400 series, and 500 series reference numbers, respectively. Use of the same second and third digits indicates like parts and analogous function within, e.g., first stage 350 and second stage 450, respectively. For example, plate member 352 and plate member 452 are structurally and functionally analogous components of first stage 350 and second stage 450, respectively. Components of cycloid gear assembly 250 may be made of any of a number of biocompatible materials as known in the art, although anodized titanium may be used in certain embodiments.

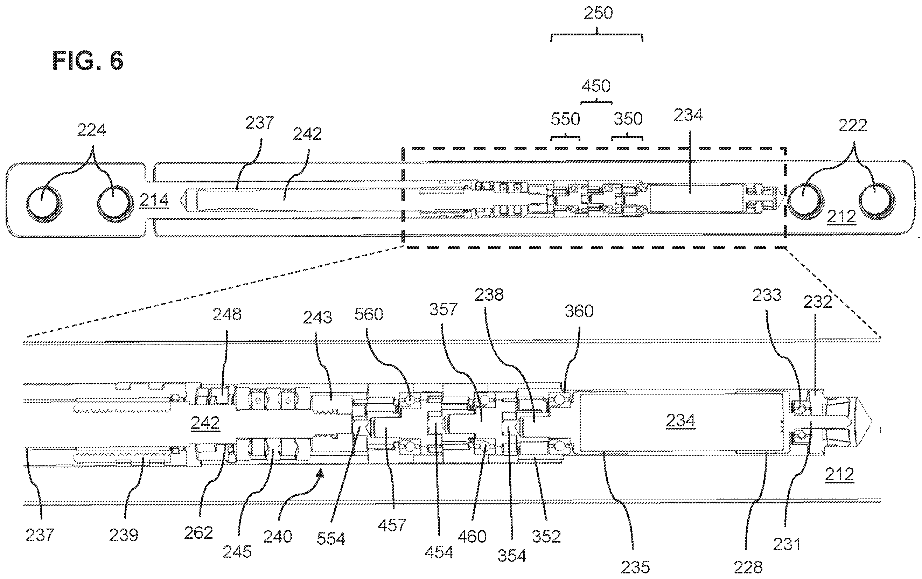

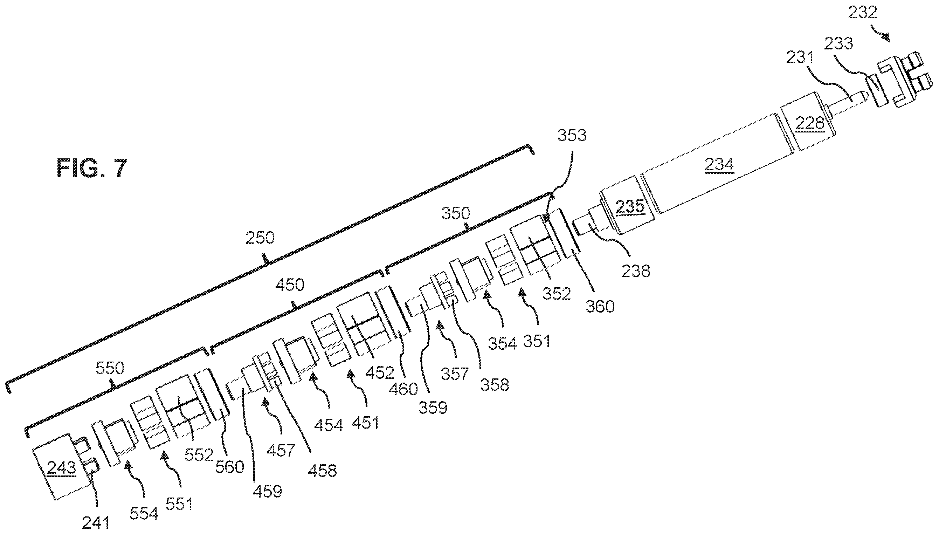

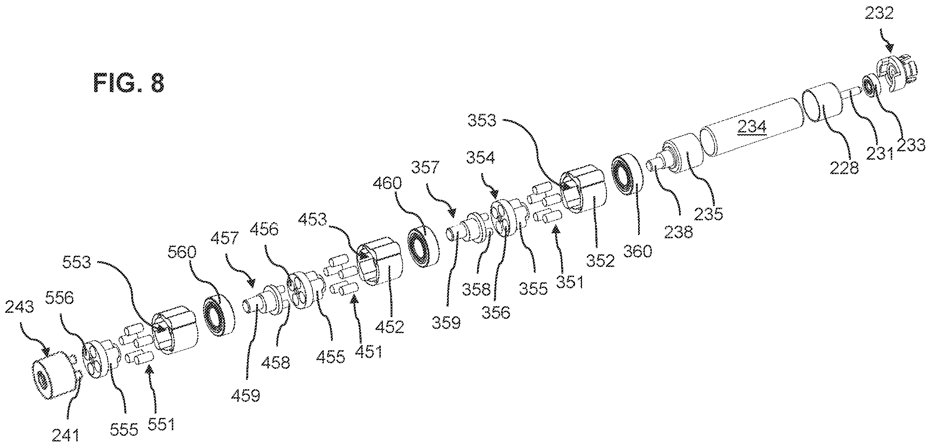

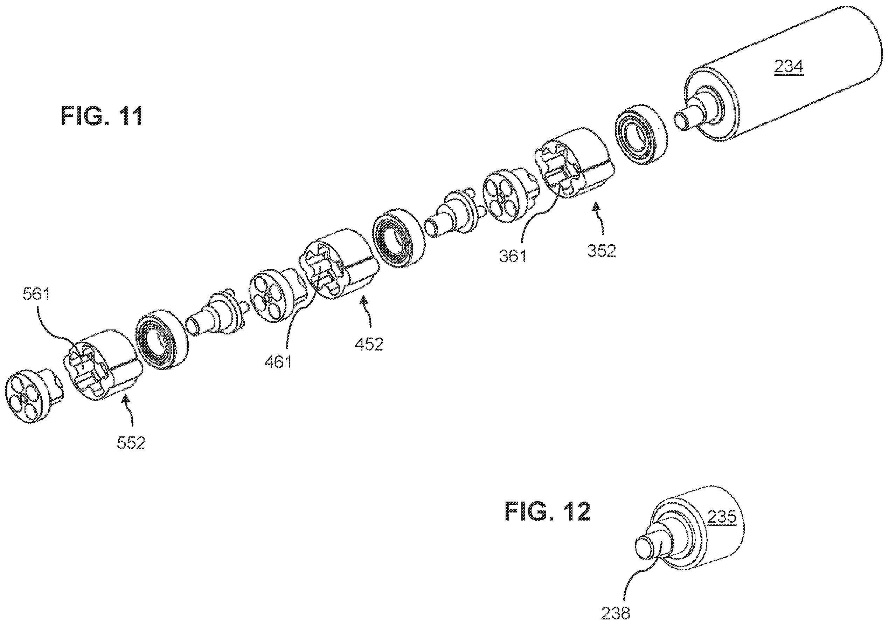

Turning to FIGS. 7-9, each stage of the cycloid gear assembly 250 may include a plurality of components. For example, first stage 350 may include a plate member 352 rotatably fixed relative to housing 212 (shown in FIGS. 5-6). Plate member 352 may have an annular opening 353 therethrough, and may include a plurality of pins 351 spaced about a periphery of the annular opening 353 (FIGS. 8-9). Pins 351 may be configured to roll freely relative to plate member 352, thereby reducing the friction generated by the system. In certain embodiments, the number of pins 351 may be five. However, other embodiments are also contemplated as part of the disclosure, in which other numbers of pins 351 are possible, for example, plate member 352 may include one pin, two pins, three pins, four pins, five pins, six pins, and so on. Additionally, the plate member 352 may be configured to incorporate the pin surfaces 361, as shown in FIG 11. In the embodiment of FIG. 11, the pin surfaces 361 prevent rolling, but facilitate greater ease of assembly. These pins 351 (FIGS. 8-9) or pin surfaces 361 on plate member 352 (FIG. 11) may by spaced evenly about a periphery of the annular opening 353 through plate member 352. Additionally, as shown in FIG. 6, a bearing 360 may be positioned within the plate member 352 adjacent the pins 351.

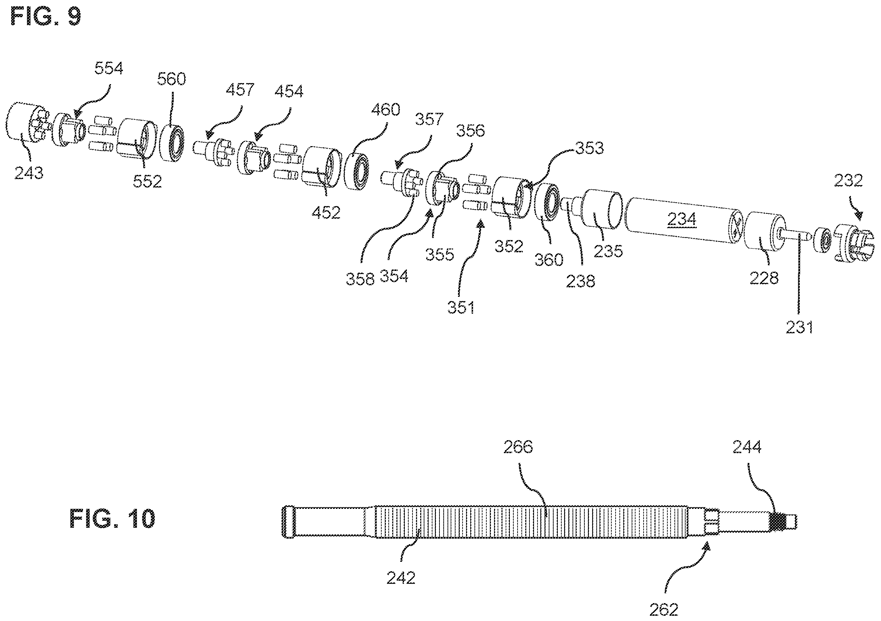

First stage 350 may further include a cycloidal eccentrically driven disc 354, having a lobed portion, which extends longitudinally and includes a plurality of lobes 355 with an axially extending annular opening therethrough (as best seen in FIG. 9). The lobed portion may be configured to be axially inserted into annular opening 353 of plate member 352. In certain embodiments, such as the embodiments depicted in FIGS. 7-9, the number of lobes 355 may be four. In some embodiments, the number of lobes 355 may be at least two. However, in other embodiments, other numbers of lobes 355 may be present, e.g., one lobe, two lobes, three lobes, four lobes, five lobes, and so on. Regardless of the number of pins 351 or lobes 355 present, the number of lobes 355 is equal to (n-1), where n is the number of pins 351 present, in order to generate a smooth epitrochoid curve. Eccentric shaft 238 on magnet cup 235 is configured to be inserted through annular opening 353 in plate member 352 from the opposite direction as the longitudinally extending lobed portion 355 of eccentrically driven disc 354, such that eccentric shaft 238 extends through annular opening 353 in plate member 352 as well as through the annular opening through cycloidal eccentrically driven disc 354. In this manner, eccentric shaft 238 of eccentric magnet cup 235 is configured to drive rotation of eccentrically driven disc 254 within plate member 352. In certain embodiments, the cycloidal eccentrically driven disc 354 may follow an epitrochoid curve during use, providing a smooth rolling load surface. Bearing 360 may be provided to support eccentric shaft 238 within plate member 352 (FIGS. 5-8).

Cycloidal eccentrically driven disc 354 may further include a plurality of recess features 356 on a face opposite the longitudinally extending lobes 355. In an embodiment comprising only a single gear stage 350 in cycloid gear assembly 250, recess features 356 are configured to be coupled to lead screw assembly 240 as discussed further below.

In other embodiments containing two or more stages 350, 450, and so on in cycloid gear assembly 250, recess features 356 are configured to engage output shaft member 357. Output shaft member 357 may include a plurality of pins 358 extending in a first direction, and an output shaft 359 extending in a second direction, opposite the first direction. Pins 358 may be configured to engage in one to one fashion with recess features 356 of cycloidal eccentrically driven disc 354. The number of pins 358 may be equivalent to the number of recess features 356 present on cycloidal eccentrically driven disc 354. In certain embodiments, the number of pins 358 and recess features 356 may be four, although other configurations are also within the scope of the invention, e.g., one pin, two pins, three pins, four pins, and so on. Output shaft 359 of output shaft member 357 may be an eccentric shaft.

Output shaft 359 may engage second gear stage 450 in the same manner in which eccentric shaft 238 of magnet cup 235 engages with plate member 352 and cycloidal eccentrically driven disc 354 in first stage 350. In particular, eccentric output shaft 359 may extend toward second gear stage 450. Second gear stage 450 may include a plate member 452 rotatably fixed relative to housing 212 (shown in FIGS. 5-6). Plate member 452 may have an annular opening 453 therethrough, and may include a plurality of pins 451 or pin surfaces 461 (FIG. 11) spaced about a periphery of the annular opening 453. In certain embodiments, the number of pins 451 or pin surfaces 461 may be five. However, other embodiments are also contemplated as part of the disclosure, in which other numbers of pins 451 or pin surfaces 461 are possible, for example, plate members 452 including one pin, two pins, three pins, four pins, five pins, six pins, and so on. These pins 451 or pin surfaces 461 may by spaced evenly about a periphery of the annular opening 453 through plate member 452. Additionally, as shown in FIG. 6, a bearing 460 may be positioned within the plate member 452 adjacent the pins 451 or pin surfaces 461.

Second stage 450 may further include a cycloidal eccentrically driven disc 454, having a longitudinally extending portion having a plurality of lobes 455 with an axially extending annular opening therethrough (as best seen in FIG. 9). The plurality of lobes may be configured to be axially inserted into annular opening 453 of plate member 452. In certain embodiments, such as the embodiments depicted in FIGS. 7-9, the number of lobes 455 may be four. However, in other embodiments, other numbers of lobes 455 may be present, e.g., one lobe, two lobes, three lobes, four lobes, five lobes, and so on. Output shaft 359 of output shaft member 357 is configured to be inserted through annular opening 453 in plate member 452 from the opposite direction as the longitudinally extending lobed portion 455 of eccentrically driven disc 454, such that output shaft 359 extends through annular opening 453 in plate member 452 as well as through the annular opening through cycloidal eccentrically driven disc 454. In this manner, output shaft 359 is configured to drive rotation of eccentrically driven disc 454 within plate member 452. In certain embodiments, the cycloidal eccentrically driven disc 454 may follow an epitrochoid curve during use, providing a smooth rolling load surface. Bearing 460 may be provided to support eccentric shaft member 357 within plate member 452 (FIG. 6).

Cycloidal eccentrically driven disc 454 may further include a plurality of recess features 456 opposite the longitudinally extending lobes 455. In an embodiment comprising two gear stages 350, 450 in cycloid gear assembly 250, recess features 456 are configured to couple to lead screw assembly 240 as discussed further below.

In other embodiments containing three or more stages 350, 450, 550, and so on in cycloid gear assembly 250, recess features 456 are configured to engage output shaft member 457. Output shaft member 457 may include a plurality of pins 458 extending in a first direction, and an output shaft 459 extending in a second direction, opposite the first direction. Pins 458 may be configured to engage in one to one fashion with recess features 456 of cycloidal eccentrically driven disc 454. The number of pins 458 may be equivalent to the number of recess features 456 present on cycloidal eccentrically driven disc 454. In certain embodiments, the number of pins 458 and recess features 456 may be four, although other configurations are also within the scope of the invention, e.g., one pin, two pins, three pins, four pins, and so on. Output shaft 459 of output shaft member 457 may be an eccentric shaft.

Output shaft 459 may engage third gear stage 550 in the same manner in which output shaft 359 engages with plate member 452 and cycloidal eccentrically driven disc 454 in second stage 450. In particular, eccentric output shaft 459 may extend toward third gear stage 550. Third gear stage 550 may include a plate member 552 rotatably fixed relative to housing 212 (shown in FIGS. 5-6). Plate member 552 may have an annular opening 553 therethrough, and may include a plurality of pins 551 or pin surfaces 561 (FIG. 11) spaced about a periphery of the annular opening 553. In certain embodiments, the number of pins 551 or pin surfaces 561 may be five. However, other embodiments are also contemplated as part of the disclosure, in which other numbers of pins 551 or pin surfaces 561 are possible, for example, plate members 552 including one pin, two pins, three pins, four pins, five pins, six pins, and so on. These pins 551 or pin surfaces 561 may by spaced evenly about a periphery of the annular opening 553 through plate member 552. Additionally, as shown in FIG. 6, a bearing 560 may be positioned within the plate member 552 adjacent the pins 551 or pin surfaces 561.

Third stage 550 may further include a cycloidal eccentrically driven disc 554, having a longitudinally extending portion having a plurality of lobes 555 with an axially extending annular opening therethrough (as best seen in FIG. 9). The plurality of lobes may be configured to be axially inserted into annular opening 553 of plate member 552. In certain embodiments, such as the embodiments depicted in FIGS. 7-9, the number of lobes 555 may be four. However, in other embodiments, other numbers of lobes 555 may be present, e.g., one lobe, two lobes, three lobes, four lobes, five lobes, and so on. Output shaft 459 of output shaft member 457 is configured to be inserted through annular opening 553 in plate member 552 from the opposite direction as the longitudinally extending lobed portion 555 of eccentrically driven disc 554, such that output shaft 459 extends through annular opening 553 in plate member 552 as well as through the annular opening through cycloidal eccentrically driven disc 554. In this manner, output shaft 459 is configured to drive rotation of eccentrically driven disc 554 within plate member 552. In certain embodiments, the cycloidal eccentrically driven disc 554 may follow an epitrochoid curve during use, providing a smooth rolling load surface. Bearing 560 may be provided to support eccentric shaft 457 within plate member 452 (FIG. 6).

Cycloidal eccentrically driven disc 554 may further include a plurality of recess features 556 opposite the longitudinally extending lobes 555. In an embodiment comprising three gear stages 350, 450, 550 in cycloid gear assembly 250, e.g., as shown in FIGS. 5-8, recess features 556 are configured to engage lead screw assembly 240 as discussed further below.

In other embodiments containing four or more stages in cycloid gear assembly 250, recess features 556 are configured to engage an output shaft member in a manner analogous to output shaft member 457, in a pattern which may continue through further stage iterations. Such an output shaft member may include a plurality of pins extending in a first direction, and an output shaft extending in a second direction, opposite the first direction. The pins may be configured to engage in one to one fashion with recess features 556 of cycloidal eccentrically driven disc 554. The number of pins may be equivalent to the number of recess features 556 present on cycloidal eccentrically driven disc 554. In certain embodiments, the number of pins and recess features 556 may be four, although other configurations are also within the scope of the invention, e.g., one pin, two pins, three pins, four pins, and so on. The output shaft of the output shaft member may be an eccentric shaft.

Regardless of the number of gear stages in cycloid gear assembly 250, the cycloidal eccentrically driven disc 354, 454, 554 of the terminal stage of gears may be configured to engage lead screw assembly 240 as mentioned above. The terminal stage of gears in the embodiment depicted in FIGS. 5-8 is third stage 550; therefore, in these embodiments, cycloidal eccentrically driven disc 554 is configured to engage lead screw assembly 240. In embodiments having one or two stages of gears, cycloidal eccentrically driven disc 354 or 454, respectively, may be configured to engage lead screw assembly 240.

Lead screw assembly 240 may include lead screw 242 (FIG. 10), which may be, e.g., hex-driven. Lead screw assembly 240 may further include an output shaft and lead screw stop 243 (FIGS. 6-9), which may include a plurality of axially extending pins 241 (FIG. 7) extending in a first direction, which are configured to engage recess features 556 in use. It is understood that in other embodiments, for example in embodiments having only one stage of gears, output shaft and lead screw stop 243 may directly engage recess features 356 in use, and in embodiments having two stages of gears, output shaft and lead screw stop 243 may engage recess features 456 in use. In any event, pins 241 may be shaped and configured substantially similarly to pins 358, 458, and so on, on output shaft members 357, 457, described previously herein.

Opposite the plurality of pins 241, a lead screw stop feature is provided, for threadably receiving an end of lead screw 242. As shown in, e.g., FIGS. 5-6, the lead screw stop feature may include a female threaded feature, configured to receive and engage a threaded end portion 244 (FIG. 10) of lead screw 242. The lead screw stop feature and pins 241 that collectively comprise output shaft and lead screw stop 243 may be unitarily formed or may include two or more components welded or otherwise adhered together as is understood within the art.

Lead screw assembly 240 further includes a bidirectional thrust bearing 245 (FIGS. 4-6) disposed about lead screw 242 and configured to support the axial load thereof. Bidirectional thrust bearing 245 is disposed adjacent to output shaft and lead screw stop 243. A retaining ring 246, which may be, e.g., a split washer, may be disposed about lead screw 242 and adjacent to bidirectional thrust bearing 245. As shown in FIGS. 4-6, lead screw 242 is coupled at one end to cycloid gear assembly 250 by output shaft and lead screw stop 243. From this coupling, lead screw 242 extends through bidirectional thrust bearing 245 and retaining ring 246, and into cavity 237 of distraction rod 214. Nut 239 is affixed to an inner surface of cavity 237, and engages a threaded portion 266 along the length of lead screw 242, as shown in FIGS. 5-6.

The use of a cycloid gear assembly to provide the gear reduction ratios described herein offers a number of advantages. Because cycloid gears do not include teeth, which present a limitation on ability to scale to small sizes, cycloid gear assemblies are readily scalable to implants designed for smaller size bones and patients, for example, jaw or finger bones. In particular, device 210 may be scaled down to provide a plate thickness or nail outer diameter of about 6.4 mm thick. With the use of bearings and/or bushings, cycloid gear assembly 250 as described herein may transmit loads with up to 96% efficiency. This efficiency also contributes to scalability, as smaller magnets and/or fewer gear stages may be used to generate the necessary torque to perform the distraction.

In certain embodiments of device 210, an anti-jam feature may be disposed about lead screw 242, at an axial position between the open end or shoulder 249 of distraction shaft 214 and cycloid gear assembly 250. In certain embodiments, one or more other components may also be disposed over lead screw 242 at an axial position(s) between the open end of distraction shaft 214 and the cycloid gear assembly 250, such that the anti-jam feature may be disposed more immediately between, e.g., the open end or shoulder 249 of distraction shaft 214, and retaining ring 246.

In embodiments that include an anti-jam feature as described herein, such as illustrated in FIGS. 4-6, the anti-jam feature may serve to reduce or avoid entirely the possibility of "bottoming out" or jamming between components, e.g., between distraction rod 214 and lead screw stop 243 in the fully retracted state. The anti-jam feature adds to the torque delivered by the driving element and gear assembly, e.g., cycloid gear assembly 250, to provides an additional spring force to overcome surface friction, thereby unjamming the components, and helping to initiate distraction of rod 214.

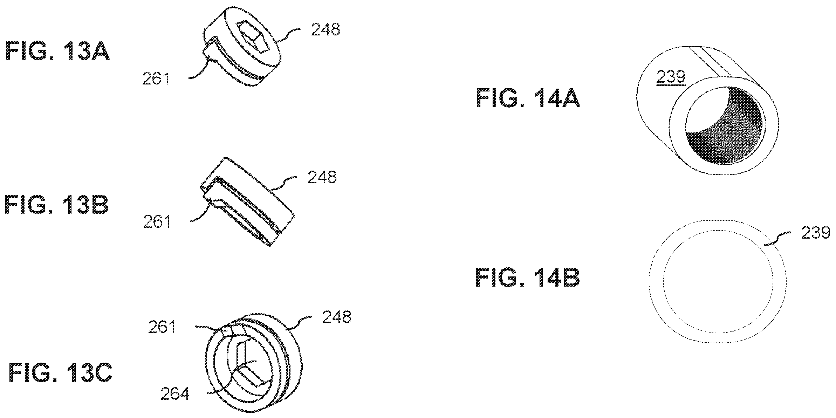

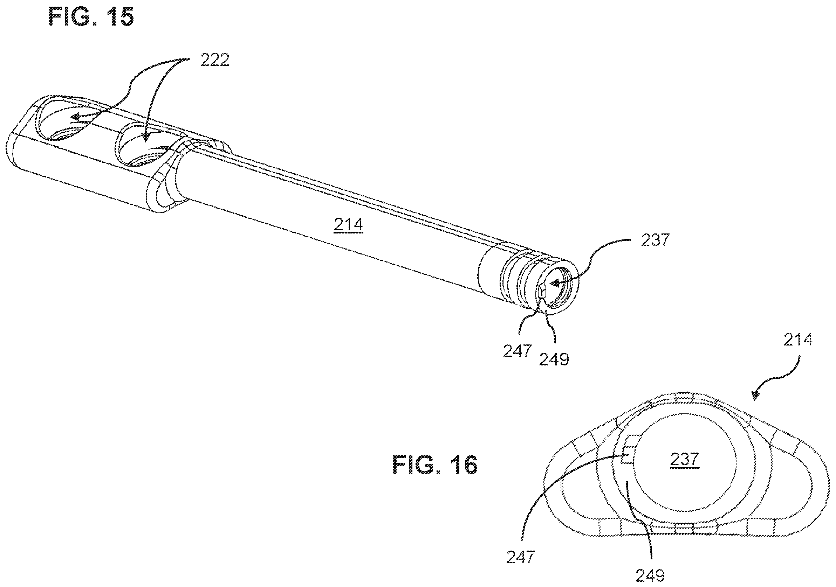

The anti-jam feature described herein may be in the form of an anti-jam spring 248, which may be a one-piece helical spring as illustrated in FIGS. 13A-13C, having a substantially round or oblong outer cross-sectional shape. In embodiments including anti-jam spring 248, distraction rod 214 may include a first tab protrusion 247 (FIGS. 4, 15, 16) extending from a shoulder 249 (FIGS. 15, 16) adjacent an open end of the internal cavity 237 of the distraction shaft 214. Tab protrusion 247 is configured to cooperate with anti-jam spring 248 as described herein.

Anti-jam spring 248 may include a second tab protrusion 261 configured to matingly engage with the first tab protrusion 247 on the distraction shaft 214. Anti-jam spring 248 may further include a keyed opening 264 (FIG. 13C) which may be, e.g., hexagonally shaped, about which the helix of the spring 248 is arranged. The shape of keyed opening 264 is configured to complement a cross sectional shape of lead screw 242 at corresponding axial position 262 (FIG. 6). In this manner, in embodiments in which lead screw 242 has a hexagonal cross-sectional shape at corresponding axial position 262, anti-jam spring 248 is configured to be hex-driven, and rotationally fixed to lead screw 242. This configuration enables anti-jam spring 248 to stay on-center relative to lead screw assembly 240 without tilting or floating, and remain constrained in a lead screw shoulder position. In use, when anti-jam spring 248 is rotated by lead screw 242, tab 261 meets and engages with distraction rod tab 247. The resistance provided by distraction rod tab 247 causes anti-jam spring 248 to open into the inner diameter of housing 212, and provide the spring force to overcome surface friction as described above. Anti-jam spring 248 is further configured to maintain engagement and face contact with shoulder 249 of distraction rod 214 during full retraction of distraction rod 214.

Anti-jam feature 248 as described herein may be used in the context of a variety of distraction devices. For example, anti-jam feature 248 may be used in conjunction with device 210 including a cycloid gear assembly 250 as described and illustrated herein. However, anti-jam feature 248 may also be used in a similar context in both intra- and extramedullary distraction and compression devices, devices having a variety of driving elements, e.g., magnetic and motor-based, and may also be used in the context of implant devices including a variety of gear assemblies such as, e.g., cycloid gear assembly 250 or planetary gear assemblies such as those described in, e.g., International Patent Application Publication No. WO/2017/132646 A1 and International Patent Application No. PCT/US2021/041701, filed July 14, 2021.

In distraction devices such as device 210, rotation of distraction rod 214 about a longitudinal axis thereof within housing 212 may be clinically undesirable. Therefore, in certain embodiments as shown in FIGS. 15-16, an anti-rotation feature may be provided in the form of a distraction rod 214 having a cross sectional shape that is oblong. In certain embodiments, the outer cross sectional shape of distraction rod 214 may be oblong, and a cross sectional shape of inner cavity 237 may be oblong. In embodiments having an oblong cross-sectional shaped inner cavity 237, nut 239 may also have an outer surface that is oblong in cross section, as shown in FIGS. 14A-14B, such that it matches the contours of the inner surface of cavity 237 of distraction rod 214. Lead screw 242 may rotate within nut 239 as described above and depicted in FIGS. 5-6, while the oblong outer shape of nut 239, oblong inner and outer shape of distraction rod 214, and oblong shape of corresponding housing 212 cooperate to maintain a fixed rotational position of housing 212, distraction rod 214, and nut 239 with respect to one another.

Oblong nut 239 and oblong distraction rod 214 as described herein may be used in the context of a variety of distraction devices. For example, oblong nut 239 and oblong distraction rod 214 may be used in conjunction with device 210 including a cycloid gear assembly 250 as described and illustrated herein. However, the cooperating oblong nut 239 and oblong distraction rod 214 may also be used in a similar context in both intra- and extramedullary distraction and compression devices, devices having a variety of driving elements, e.g., magnetic and motor-based, and may also be used in the context of implant devices including a variety of gear assemblies such as, e.g., cycloid gear assembly 250 or planetary gear assemblies such as those described in, e.g., International Patent Application Publication No. WO/2017/132646 A1 and International Patent Application No. PCT/US2021/041701, filed July 14, 2021. The cooperating oblong nut 239 and oblong distraction rod 214 may also be used in device embodiments in combination with anti-jam spring 248. In such embodiments, anti-jam spring 248 may also have an oblong outer cross sectional shape, similar to that of nut 239. In other embodiments, the cooperating oblong nut 239 and oblong distraction rod 214 may be used in devices that do not include an anti-jam spring 248.

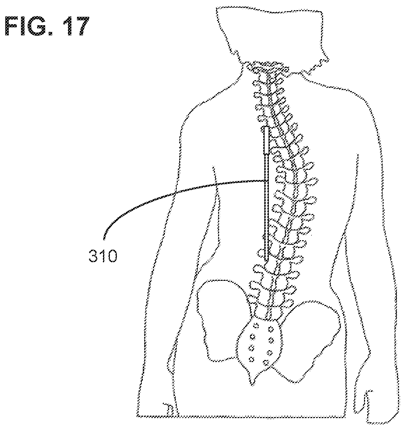

While the present invention has been shown and described relative to extramedullary limb lengthening devices, it is to be understood that embodiments herein may also be used for intramedullary distracting devices. Further, as described herein, the device is capable of both distraction and retraction, and therefore, it is to be understood that the present invention can also be used for compression of bones, e.g., in correction of non-unions. Even further, embodiments described herein can also be used for correcting a curvature of a spine, and thus can be configured as an adjustable spinal rod 310 (FIG. 17) that can be coupled to vertebra of a spine, e.g., via pedicle screws. For example, in the embodiment of FIG. 17, in lieu of holes 222 and 224 in housing 212 and distraction rod 214 (FIGS. 2-3), the ends of device 310 may be affixed to first and second sections of bone by positioning each end within a tulip or receiver of a pedicle screw.

As used herein, the terms "first," "second," and the like, do not denote any order, quantity, or importance, but rather are used to distinguish one element from another, and the terms "a" and "an" herein do not denote a limitation of quantity, but rather denote the presence of at least one of the referenced item. The modifier "about" used in connection with a quantity is inclusive of the stated value and has the meaning dictated by the context (e.g., includes the degree of error associated with measurement of the particular quantity). The suffix "(s)" as used herein is intended to include both the singular and the plural of the term that it modifies, thereby including one or more of that term (e.g., the metal(s) includes one or more metals). Ranges disclosed herein are inclusive and independently combinable (e.g., ranges of "up to about 25 mm, or, more specifically, about 5 mm to about 20 mm," is inclusive of the endpoints and all intermediate values of the ranges of "about 5 mm to about 25 mm," etc.).

While various embodiments are described herein, it will be appreciated from the specification that various combinations of elements, variations or improvements therein may be made by those skilled in the art, and are within the scope of the invention. In addition, many modifications may be made to adapt a particular situation or material to the teachings of the invention without departing from essential scope thereof. Therefore, it is intended that the invention not be limited to the particular embodiment disclosed as the best mode contemplated for carrying out this invention, but that the invention will include all embodiments falling within the scope of the appended claims.

## Claims

1. A device (210) configured for placement between a first section (102) of a bone and a second section (104) of the bone, the device (210) comprising:
a distraction shaft (214) having an internal cavity (237) disposed therein, the distraction shaft (214) being configured for fixation to the first section (102) of bone;
a housing (212) configured for fixation to the second bone section (104), wherein the distraction shaft (214) is configured to be axially movable relative to, and disposed partially within the housing (212);
a driving element (234) disposed within the housing (212);
a cycloid gear assembly (250) configured to be rotatably driven by the driving element (234); and
a lead screw assembly (240) disposed at least partly within the internal cavity of the distraction shaft (214), the lead screw assembly (242) being configured to rotatably advance and/or retract a lead screw (242) within the internal cavity (237), and to be rotatably driven by the cycloid gear assembly (250), wherein the cycloid gear assembly (250) further comprises one or more cycloid gear stages (350, 450, 550), **characterized in that**
each stage (350, 450, 550) of the one or more cycloid gear stages (350, 450, 550) has a gear reduction ratio of 4:1 and **in that**
the device further comprises a first tab protrusion (247) extending from a shoulder (249) adjacent an open end of the internal cavity (237) of the distraction shaft (214) and an anti-jam feature (248) disposed over the lead screw (242), between the open end of the distraction shaft (214) and the cycloid gear assembly (250) wherein the anti-jam feature comprises a helical spring (248) having second tab protrusion (261) configured to matingly engage with the first tab protrusion (247) on the distraction shaft (214).

2. The device of claim 1, wherein each stage (350, 450, 550) of the one or more cycloid gear stages further comprises:
a plate member (352, 452, 552) rotatably fixed relative to the housing (212) and having an annular opening (353, 453, 553) therethrough, the plate member (352, 452, 552) having five pins (351, 451, 551) evenly spaced about a periphery of the annular opening (353, 453, 553);
a cycloidal eccentrically driven disc (354, 454, 554) having a longitudinally extending portion having four lobes (355, 455, 555), the four lobes (355, 455, 555) being configured to be inserted into the annular opening (353, 453, 553), wherein the cycloidal eccentrically driven disc (354, 454, 554) further comprises a plurality of recess features (356, 456, 556) configured to matingly engage with a corresponding plurality of pins (358, 458, 558); and
an output shaft member (357, 457, 557) comprising a plurality of pins (358, 458, 558) configured to matingly engage with the plurality of recess features (356, 456, 556), and an output shaft (359, 459, 559) extending in a direction opposite that of the plurality of pins (358, 458, 558).

3. The device of claim 2, wherein the cycloidal eccentrically driven disc (354, 454, 554) further comprises an epitrochoid curve.

4. The device of claim 1, wherein the one or more cycloid gear stages (350, 450, 550) further comprises two cycloid gear stages and wherein preferably the output shaft (359) of the first stage (350) is an eccentric shaft, and the output shaft (459) of the second stage is coupled to the lead screw (242).

5. The device of claim 1, wherein the one or more cycloid gear stages (350, 450, 550) further comprises three or more cycloid gear stages (350, 450, 550) and wherein preferably the output shaft (359) of the first stage (350 and the output shaft (459) of the second stage (450) are eccentric shafts, and the output shaft (559) of the third stage (550) is coupled to the lead screw (242).

6. The device of claim 1, further comprising a coupling member (235) having an eccentric shaft (238) extending therefrom,
wherein the coupling member (235) is configured to couple the driving element (234) to the cycloid gear assembly (250) and is further configured to be rotated by the driving element (234), and, using the eccentric shaft (238), to rotatably drive the cycloid gear assembly (250).

7. The device of claim 6, further comprising a bearing (360) configured to support the eccentric shaft (238) of the coupling member (235).

8. The device of claim 1, wherein the driving element (234) further comprises a permanent magnet (234) disposed within and configured to be rotatable relative to the housing (212).

9. The device of claim 8, wherein rotation of the permanent magnet (234) causes rotation of the cycloid gear assembly (250) and the lead screw (242) relative to the housing (212) and the distraction shaft (214), thereby producing axial movement of the lead screw (242) within the internal cavity (237), and thereby lengthening or shortening the device (210).

10. The device of claim 8 or 9 and further comprising a cap (228) having a centric spindle (231) extending therefrom and a keeper (232) and a bearing (233) cooperating with one another and with the spindle (231) to support the permanent magnet (234) within the housing (212).

11. The device of claim 10 and further comprising a coupling member in the form of a cup (235) on the opposite end of permanent magnet (234) including an eccentric shaft (238) extending therefrom in a direction opposite spindle (231), and toward the lead screw (242) and the distraction shaft (214), the cap (235) with eccentric shaft (238) being configured to couple the permanent magnet (234) to the gear assembly.

12. The device of claim 1, wherein the driving element (234) further comprises a motor.

13. The device of claim 1, wherein the internal cavity (237) further comprises a nut (239) affixed to an inner surface thereof, the nut (239) being configured to engage with the lead screw (242).

14. The device of claim 1, wherein the distraction shaft (214) has an oblong cross sectional shape.

15. The device of claim 14, wherein the anti-jam feature (248) has an outer cross sectional shape that is one of substantially circular or oblong, and
wherein the anti-jam feature (248) further comprises an opening (264) having a keyed shape that is complementary to a cross sectional shape of the lead screw (242) at a corresponding axial position, such that the anti-jam feature (248) is rotationally fixed relative to the lead screw (242) when engaged to prevent jamming, wherein the keyed shape (264) is preferably hexagonal.

## Patentansprüche

1. Vorrichtung (210), die zur Anordnung zwischen einem ersten Abschnitt (102) eines Knochens und einem zweiten Abschnitt (104) des Knochens eingerichtet ist, wobei die Vorrichtung (210) aufweist:
- eine Distraktionswelle (214) mit einem darin angeordneten inneren Hohlraum (237), wobei die Distraktionswelle (214) zur Fixierung am ersten Knochenabschnitt (102) eingerichtet ist;
- ein Gehäuse (212), das zur Fixierung am zweiten Knochenabschnitt (104) eingerichtet ist, wobei die Distraktionswelle (214) eingerichtet ist, um relativ zum Gehäuse (212) axial beweglich zu sein und teilweise innerhalb diesem angeordnet ist;
- ein Antriebselement (234), das im Gehäuse (212) angeordnet ist;
- eine Zykloidengetriebeanordnung (250), die eingerichtet ist, um durch das Antriebselement (234) drehbar angetrieben zu werden; und
- eine Leitspindelanordnung (240), die zumindest teilweise im inneren Hohlraum der Distraktionswelle (214) angeordnet ist, wobei die Leitspindelanordnung (242) eingerichtet ist, um eine Leitspindel (242) im inneren Hohlraum (237) drehbar vorzuschieben und/oder zurückzuziehen und um durch die Zykloidengetriebeanordnung (250) drehbar angetrieben zu werden, wobei die Zykloidengetriebeanordnung (250) ferner eine oder mehrere Zykloidengetriebestufen (350, 450, 550) aufweist, **dadurch gekennzeichnet, dass**
- jede Stufe (350, 450, 550) einer oder mehrerer Zykloidengetriebestufen (350, 450, 550) ein Untersetzungsverhältnis von 4:1 aufweist, und dass
- die Vorrichtung ferner einen ersten Laschenvorsprung (247) aufweist, der sich von einer Schulter (249) neben einem offenen Ende des inneren Hohlraums (237) der Distraktionswelle (214) und einer Blockiereinrichtung (248), die über der Leitspindel (242) angeordnet ist, zwischen dem offenen Ende der Distraktionswelle (214) und der Zykloidengetriebeanordnung (250) erstreckt, wobei die Blockiereinrichtung eine Schraubenfeder (248) mit einem zweiten Laschenvorsprung (261) aufweist, der eingerichtet ist, um passend mit dem ersten Laschenvorsprung (247) an der Distraktionswelle (214) in Eingriff zu kommen.

2. Vorrichtung nach Anspruch 1, wobei jede Stufe (350, 450, 550) ferner eine oder mehrere Zykloidengetriebestufen aufweist:
- ein Plattenelement (352, 452, 552), das relativ zum Gehäuse (212) drehbar fixiert ist und eine ringförmige Öffnung (353, 453, 553) aufweist, wobei das Plattenelement (352, 452, 552) fünf Stifte (351, 451, 551) aufweist, die gleichmäßig um einen Umfang der ringförmigen Öffnung (353, 453, 553) beabstandet sind;
- eine zykloide, exzentrisch angetriebene Scheibe (354, 454, 554) mit einem sich in Längsrichtung erstreckenden Abschnitt mit vier Nocken (355, 455, 555), wobei die vier Nocken (355, 455, 555) eingerichtet sind, um in die ringförmige Öffnung (353, 453, 553) eingesetzt zu werden, wobei die zykloide, exzentrisch angetriebene Scheibe (354, 454, 554) ferner eine Mehrzahl von Aussparungseinrichtungen (356, 456, 556) aufweist, die eingerichtet sind, um passend mit einer entsprechenden Mehrzahl von Stiften (358, 458, 558) in Eingriff zu kommen; und
- ein Ausgangswellenelement (357, 457, 557) mit einer Mehrzahl von Stiften (358, 458, 558), die eingerichtet sind, um passend mit der Mehrzahl von Aussparungseinrichtungen (356, 456, 556) in Eingriff zu kommen, und einer Ausgangswelle (359, 459, 559), die sich in eine Richtung erstreckt, die der der Mehrzahl von Stiften (358, 458, 558) entgegengesetzt ist.

3. Vorrichtung nach Anspruch 2, wobei die zykloide, exzentrisch angetriebene Scheibe (354, 454, 554) ferner eine epitrochoide Kurve aufweist.

4. Vorrichtung nach Anspruch 1, wobei eine oder mehrere Zykloidengetriebestufen (350, 450, 550) ferner zwei Zykloidengetriebestufen aufweisen und wobei vorzugsweise die Ausgangswelle (359) der ersten Stufe (350) eine Exzenterwelle ist und die Ausgangswelle (459) der zweiten Stufe mit der Leitspindel (242) verbunden ist.

5. Vorrichtung nach Anspruch 1, wobei eine oder mehrere Zykloidengetriebestufen (350, 450, 550) ferner drei oder mehr Zykloidengetriebestufen (350, 450, 550) aufweisen und wobei vorzugsweise die Ausgangswelle (359) der ersten Stufe (350 und die Ausgangswelle (459) der zweiten Stufe (450) Exzenterwellen sind und die Ausgangswelle (559) der dritten Stufe (550) mit der Leitspindel (242) verbunden ist.

6. Vorrichtung nach Anspruch 1, die ferner ein Kupplungselement (235) mit einer davon ausgehenden Exzenterwelle (238) aufweist,
- wobei das Kupplungselement (235) eingerichtet ist, um das Antriebselement (234) mit der Zykloidengetriebeanordnung (250) zu verbinden und ferner eingerichtet ist, um durch das Antriebselement (234) gedreht zu werden und um unter Verwendung der Exzenterwelle (238) die Zykloidengetriebeanordnung (250) drehbar anzutreiben.

7. Vorrichtung nach Anspruch 6, die ferner ein Lager (360) aufweist, das eingerichtet ist, um die Exzenterwelle (238) des Kupplungselements (235) zu lagern.

8. Vorrichtung nach Anspruch 1, wobei das Antriebselement (234) ferner einen Permanentmagneten (234) aufweist, der innerhalb des Gehäuses (212) angeordnet ist, und eingerichtet ist, um relativ dazu drehbar zu sein.

9. Vorrichtung nach Anspruch 8, wobei eine Drehung des Permanentmagneten (234) eine Drehung der Zykloidengetriebeanordnung (250) und der Leitspindel (242) relativ zum Gehäuse (212) und der Distraktionswelle (214) bewirkt, wodurch eine axiale Bewegung der Leitspindel (242) innerhalb des inneren Hohlraums (237) erzeugt wird und dadurch die Vorrichtung (210) verlängert oder verkürzt wird.

10. Vorrichtung nach Anspruch 8 oder 9, die ferner eine Kappe (228) mit einer davon ausgehenden Zentrierspindel (231) sowie einen Halter (232) und ein Lager (233) aufweist, die miteinander und mit der Spindel (231) zusammenwirken, um den Permanentmagneten (234) im Gehäuse (212) zu lagern.

11. Vorrichtung nach Anspruch 10, die ferner ein Kupplungselement in Form eines Bechers (235) am gegenüberliegenden Ende des Permanentmagneten (234) mit einer Exzenterwelle (238) aufweist, die sich von diesem in eine Richtung entgegengesetzt zur Spindel (231) und in Richtung der Leitspindel (242) und der Distraktionswelle (214) erstreckt, wobei die Kappe (235) mit Exzenterwelle (238) eingerichtet ist, um den Permanentmagneten (234) mit der Getriebeanordnung zu verbinden.

12. Vorrichtung nach Anspruch 1, wobei das Antriebselement (234) ferner einen Motor aufweist.

13. Vorrichtung nach Anspruch 1, wobei der innere Hohlraum (237) ferner eine Mutter (239) aufweist, die an einer Innenfläche davon fixiert ist, wobei die Mutter (239) eingerichtet ist, um mit der Leitspindel (242) in Eingriff zu kommen.

14. Vorrichtung nach Anspruch 1, wobei die Distraktionswelle (214) eine längliche Querschnittsform aufweist.

15. Vorrichtung nach Anspruch 14, wobei die Blockiereinrichtung (248) eine äußere Querschnittsform aufweist, die im Wesentlichen kreisförmig oder länglich ist, und
- wobei die Blockiereinrichtung (248) ferner eine Öffnung (264) mit einer Keilform aufweist, die komplementär zu einer Querschnittsform der Leitspindel (242) an einer entsprechenden axialen Position ist, so dass die Blockiereinrichtung (248) relativ zur Leitspindel (242) im eingerückten Zustand drehbar fixiert ist, um ein Blockieren zu verhindern, wobei die Keilform (264) vorzugsweise sechseckig ist.

## Revendications

1. Dispositif (210) configuré pour être placé entre une première section (102) d'un os et une seconde section (104) de l'os, le dispositif (210) comprenant :
une tige de distraction (214) ayant une cavité interne (237) disposée à l'intérieur, la tige de distraction (214) étant configurée pour être fixée à la première section (102) de l'os ;
un boîtier (212) configuré pour être fixé à la seconde section osseuse (104), dans lequel la tige de distraction (214) est configurée pour être axialement mobile par rapport au boîtier (212) et disposée partiellement à l'intérieur de celui-ci ;
un élément d'entraînement (234) disposé à l'intérieur du boîtier (212) ;
un ensemble d'engrenage cycloïde (250) configuré pour être entraîné en rotation par l'élément d'entraînement (234) ; et
un ensemble de vis-mère (240) disposé au moins en partie dans la cavité interne de la tige de distraction (214), l'ensemble de vis-mère (242) étant configuré pour faire avancer et/ou rétracter de manière rotative une vis-mère (242) dans la cavité interne (237), et pour être entraîné de manière rotative par l'ensemble d'engrenage cycloïde (250), dans lequel l'ensemble d'engrenage cycloïde (250) comprend en outre un ou plusieurs étages d'engrenage cycloïde (350, 450, 550), **caractérisé en ce que**
chaque étage (350, 450, 550) d'un ou plusieurs étages d'engrenages cycloïdes (350, 450, 550) a un rapport de réduction de 4:1 et **en ce que**
le dispositif comprend en outre une première saillie de languette (247) s'étendant à partir d'un épaulement (249) adjacent à une extrémité ouverte de la cavité interne (237) de la tige de distraction (214) et un dispositif anti-blocage (248) disposé sur la vis-mère (242), entre l'extrémité ouverte de la tige de distraction (214) et l'ensemble d'engrenage cycloïde (250), dans lequel le dispositif anti-blocage comprend un ressort hélicoïdal (248) ayant une seconde saillie de languette (261) configurée pour s'accoupler avec la première saillie de languette (247) sur la tige de distraction (214).

2. Dispositif selon la revendication 1, dans lequel chaque étage (350, 450, 550) d'un ou plusieurs étages d'engrenages cycloïdes comprend en outre :
un élément de plaque (352, 452, 552) fixé de manière rotative par rapport au boîtier (212) et ayant une ouverture annulaire (353, 453, 553) à travers celui-ci, l'élément de plaque (352, 452, 552) ayant cinq broches (351, 451, 551) régulièrement espacées sur une périphérie de l'ouverture annulaire (353, 453, 553) ;
un disque cycloïdal à entraînement excentrique (354, 454, 554) ayant une partie s'étendant longitudinalement avec quatre lobes (355, 455, 555), les quatre lobes (355, 455, 555) étant configurés pour être insérés dans l'ouverture annulaire (353, 453, 553), le disque cycloïdal à entraînement excentrique (354, 454, 554) comprenant en outre une pluralité de caractéristiques d'évidement (356, 456, 556) configurées pour s'accoupler avec une pluralité correspondante de broches (358, 458, 558) ; et
un élément d'arbre de sortie (357, 457, 557) comprenant une pluralité de broches (358, 458, 558) configurées pour s'accoupler avec la pluralité de caractéristiques d'évidement (356, 456, 556), et un arbre de sortie (359, 459, 559) s'étendant dans une direction opposée à celle de la pluralité de broches (358, 458, 558).

3. Dispositif selon la revendication 2, dans lequel le disque cycloïdal à entraînement excentrique (354, 454, 554) comprend en outre une courbe épitrochoïde.

4. Dispositif selon la revendication 1, dans lequel le ou les étages d'engrenages cycloïdes (350, 450, 550) comprennent en outre deux étages d'engrenages cycloïdes et dans lequel, de préférence, l'arbre de sortie (359) du premier étage (350) est un arbre excentrique, et l'arbre de sortie (459) du deuxième étage est couplé à la vis-mère (242).

5. Dispositif selon la revendication 1, dans lequel le ou les étages d'engrenages cycloïdes (350, 450, 550) comprennent en outre trois étages d'engrenages cycloïdes ou plus (350, 450, 550) et dans lequel, de préférence, l'arbre de sortie (359) du premier étage (350 et l'arbre de sortie (459) du deuxième étage (450) sont des arbres excentriques, et l'arbre de sortie (559) du troisième étage (550) est couplé à la vis-mère (242).

6. Dispositif selon la revendication 1, comprenant en outre un élément de couplage (235) ayant un arbre excentrique (238) s'étendant à partir de celui-ci, dans lequel l'élément de couplage (235) est configuré pour coupler l'élément d'entraînement (234) à l'ensemble d'engrenage cycloïde (250) et est en outre configuré pour être entraîné en rotation par l'élément d'entraînement (234) et, à l'aide de l'arbre excentrique (238), pour entraîner en rotation l'ensemble d'engrenage cycloïde (250).

7. Dispositif selon la revendication 6, comprenant en outre un palier (360) configuré pour supporter l'arbre excentrique (238) de l'élément de couplage (235).

8. Dispositif selon la revendication 1, dans lequel l'élément d'entraînement (234) comprend en outre un aimant permanent (234) disposé à l'intérieur du boîtier (212) et configuré pour être rotatif par rapport à celui-ci.

9. Dispositif selon la revendication 8, dans lequel la rotation de l'aimant permanent (234) entraîne la rotation de l'ensemble d'engrenage cycloïde (250) et de la vis-mère (242) par rapport au boîtier (212) et à la tige de distraction (214), produisant ainsi un mouvement axial de la vis-mère (242) dans la cavité interne (237), et allongeant ou raccourcissant ainsi le dispositif (210).

10. Dispositif selon la revendication 8 ou 9 et comprenant en outre un capuchon (228) ayant un axe (231) centré s'étendant à partir de celui-ci et une pièce de maintien (232) et un palier (233) coopérant l'un avec l'autre et avec l'axe (231) pour soutenir l'aimant permanent (234) à l'intérieur du boîtier (212).

11. Dispositif selon la revendication 10 et comprenant en outre un élément de couplage sous la forme d'un capuchon (235) à l'extrémité opposée de l'aimant permanent (234) comprenant un arbre excentrique (238) s'étendant à partir de celui-ci dans une direction opposée à l'axe (231), et vers la vis-mère (242) et la tige de distraction (214), le capuchon (235) avec l'arbre excentrique (238) étant configuré pour coupler l'aimant permanent (234) à l'ensemble d'engrenage.

12. Dispositif selon la revendication 1, dans lequel l'élément d'entraînement (234) comprend en outre un moteur.

13. Dispositif selon la revendication 1, dans lequel la cavité interne (237) comprend en outre un écrou (239) fixé à sa surface intérieure, l'écrou (239) étant configuré pour s'engager dans la vis-mère (242).

14. Dispositif selon la revendication 1, dans lequel la tige de distraction (214) a une forme de section transversale oblongue.

15. Dispositif selon la revendication 14, dans lequel le dispositif anti-blocage (248) a une forme de section transversale extérieure qui est l'une des formes suivantes : sensiblement circulaire ou oblongue, et
dans lequel le dispositif anti-blocage (248) comprend en outre une ouverture (264) ayant une forme clavetée qui est complémentaire d'une forme de section transversale de la vis-mère (242) à une position axiale correspondante, de sorte que le dispositif anti-blocage (248) est fixé de manière rotative par rapport à la vis-mère (242) lorsqu'il est engagé pour empêcher le blocage, dans lequel la forme clavetée (264) est de préférence hexagonale.
